# DEMANDE DE BREVET EUROPEEN

(11) **EP 1 352 893 A1**
(43) Date de publication de la demande: **15.10.2003**
(21) Numéro de dépôt: 03290843.6
(22) Date de dépôt: 04.04.2003
(51) Int. Cl.: C07C 67/03, C11C 3/04, C07C 69/24, C07C 69/52

(54) **Procédé de production d'esters alkyliques à partir d'une huile végétale ou animale et d'un monoalcool aliphatique**

(30) Priorité: 11.04.2002 FR 0204565
(71) Demandeur: Institut Français du Pétrole, 92852 Rueil-Malmaison Cedex (FR)
(72) Inventeur: Bournay, Laurent, 69003 Lyon (FR); Hillion, Gérard, 95220 Herblay (FR); Boucot, Pierre, 69360 Ternay (FR); Chodroge, Jean-Alain, 92160 Antony (FR); Bronner, Charles, 69540 Irigny (FR); Forestiere, Alain, 69390 Vernaison (FR)

(57) **Abrégé**

On produit des esters alkyliques d'acides gras et de la glycérine à haute pureté en mettant en oeuvre un procédé comprenant un ensemble de réactions de transestérification entre une huile végétale ou animale et un monoalcool aliphatique qui utilisent un catalyseur hétérogène, par exemple à base d'aluminate de zinc, la teneur en eau dans le milieu réactionnel étant contrôlée à une valeur inférieure à une valeur limite donnée.

## Description

L'invention concerne la fabrication d'esters alkyliques dérivés d'huiles végétales ou animales, en particulier d'esters méthyliques dérivés d'huile de colza.

L'utilisation des esters méthyliques d'huiles végétales ("EMVH") comme carburants de substitution au gazole est promise à un large développement dans les deux prochaines décennies. En effet, la présence dans ces produits de monoglycérides sous forme de traces (0,8 % en masse maximum selon la norme en vigueur), permet de compenser avantageusement la perte de pouvoir lubrifiant due notamment à la réduction de la teneur en soufre dans le gazole. La teneur en soufre sera réglementée à 50 ppm masse en 2005 et 10 ppm masse en 2008. Par ailleurs, la Commission européenne a adopté un plan d'action et deux propositions de directive en vue d'encourager l'utilisation des carburants de substitution dans le secteur des transports, en commençant par des mesures réglementaires et fiscales destinées à promouvoir les biocarburants. Le plan d'action définit une stratégie permettant de remplacer, d'ici à 2020, 20 % du carburant diesel et de l'essence par des carburants de substitution dans le secteur des transports routiers. Une des directives proposées prévoit que les biocarburants représentent une proportion minimale 2 % de l'ensemble des carburants vendus à partir de 2005, proportion minimale qui atteindrait 5,75 % en 2010. La production d'esters méthyliques dérivés d'huiles végétales (désignées usuellement par "Biodiesel"), essentiellement à partir d'huile de colza, dépasse les 300 000 t/an en France. De plus, il existe d'autres applications possibles de ces produits, telles que les solvants écologiques et des composés de base pour la fabrication de sulfonates d'alcool gras, d'amides, de dimères d'esters, etc.

L'invention a pour objet un procédé de fabrication d'esters alkyliques dérivés d'huiles végétales ou animales, en particulier d'esters méthyliques dérivés d'huile de colza, qui présente l'avantage d'avoir un rendement très proche de 100%, de produire une glycérine très pure, exempte de sels et enfin qui ne produit aucun rejet en marche normale.

Des procédés de fabrication d'esters alkyliques (par exemple méthyliques) ont déjà été développés. Ils utilisent les voies classiques de la catalyse homogène avec des catalyseurs solubles comme la soude ou le méthylate de sodium par réaction d'une huile neutre avec un alcool comme le méthanol. On peut citer comme représentatif de ce type de procédé, le procédé décrit dans la demande de brevet DE-A-4 123 928 (= EP-A-0 523 767), qui implique la mise en oeuvre continue d'un catalyseur homogène basique.

Ce type de procédé présente plusieurs inconvénients. Une fois la réaction terminée, il faut neutraliser l'excès de catalyseur présent essentiellement dans la phase glycérine sous forme d'alcoolates et de savons, puis éliminer l'eau et le monoalcool (méthanol) par évaporation. Le monoalcool (méthanol) évaporé doit souvent être distillé. Pour la fraction ester, on élimine les traces de composés alcalins par lavage à l'eau et séchage.

De manière générale, dans les procédés utilisant une technique de catalyse homogène, pour atteindre les spécifications recherchées pour la glycérine et pour l'ester, il faut mettre en oeuvre une chaîne de traitements complexe et laborieuse, qui ne permet pas d'obtenir une glycérine totalement exempte de traces de sels alcalins, ce qui diminue fortement le prix de reprise de ce coproduit à haute valeur ajoutée.

D'autres procédés mettent en oeuvre des catalyseurs hétérogènes tel que celui décrit dans la demande de brevet EP-A-0 924 185. Il s'agit d'un procédé en trois étapes utilisant la catalyse hétérogène :
- une première étape (a), consiste à faire réagir une huile végétale avec un excès de monoalcool en présence d'un catalyseur hétérogène, suivi d'une élimination du monoalcool en excès et d'une séparation de la glycérine. Cette étape produit un ester brut contenant des monoglycérides résiduels ;
- une seconde étape (b), dans laquelle on soumet l'ester brut ainsi obtenu à une réaction de ré-estérification des mono-glycérides résiduels vers des di- et triglycérides, en présence d'un catalyseur hétérogène ; et
- une troisième étape (c), dans laquelle on procède à une évaporation sous pression réduite de l'ester avec recyclage du résidu d'évaporation vers l'huile de départ de l'étape (a).

L'inconvénient principal d'un tel procédé est le coût économique très important que représente la distillation sous vide de toute la production. De plus, la présence d'un recyclage représente également un surcoût. Enfin, des expériences montrent que, même sous pression très réduite, la température de fond de la colonne d'évaporation de l'ester est importante, ce qui entraîne un risque sérieux de dégradation du résidu. Ce dernier ne peut donc être totalement recyclé, il doit périodiquement être purgé, ce qui pénalise le rendement du procédé.

Enfin, le brevet FR-B-2 752 242 (= brevet US-A-5 908 946) décrit un procédé de fabrication d'au moins un ester alkylique d'acide gras et de glycérine à haut degré de pureté à partir d'une huile végétale et d'un monoalcool aliphatique en présence, par exemple, d'un catalyseur comprenant de l'aluminate de zinc, sans préciser l'enchaînement des étapes unitaires de façon détaillée. Il décrit un système incluant les étapes suivantes :
- une transestérification de l'huile en discontinu ou en continu sur un lit fixe ou dans un autoclave, avec au moins 80 - 85 % ou, de préférence, au moins 90 - 95 % de conversion ;
- une première évaporation du mono-alcool en excès ;
- une décantation de la glycérine et de l'ester, ledit ester étant recyclé dans une deuxième étape pour subir une transestérification avec une partie du mono-alcool récupéré dans la première évaporation ;
- une seconde évaporation du monoalcool ; et
- une décantation à froid et une séparation de la glycérine et de l'ester alkylique.

Par ailleurs, ce brevet mentionne que la présence d'eau est néfaste car elle favorise la formation d'acides gras, donc de réactifs qui risquent de réagir pour former des savons. Il n'enseigne pas la manière de limiter cette teneur en eau qui, en fait, constitue un problème beaucoup plus important que celui mentionné. En effet, l'eau est un inhibiteur du catalyseur et sa teneur dans le milieu réactionnel n'est pas souhaitée au-delà de 1500 ppm masse, et préférentiellement au-delà de 1000 ppm.

La présente invention permet de satisfaire cette contrainte par la mise en oeuvre en divers endroits du procédé, de séparations eau/méthanol, qui vont permettre de contrôler la teneur maximale en eau dans les zones réactionnelles, mais aussi de délivrer une glycérine de très haut niveau de pureté. Ce dernier point a un impact très important sur l'économie du procédé, puisque le prix de la glycérine dépend essentiellement de son niveau de pureté. Pour la glycérine raffinée (pureté de 96 % à 99,7 %) les prix moyennés entre 1998 et 1990 sont de 1,44 $/kg (1,64 euros/kg) en Europe, et de 1,8 $/kg (2,05 euros/kg) aux États-Unis.

D'une manière générale, le procédé de l'invention comprend la mise en oeuvre d'une réaction de transestérification d'une huile végétale (le plus souvent l'huile de colza) ou d'une huile d'origine animale telle que le suif, au moyen d'un monoalcool aliphatique (généralement le méthanol), utilisé en excès, avec séparation et recyclage de l'excès de monoalcool aliphatique, pour donner de la glycérine et de l'ester alkylique en présence d'un catalyseur hétérogène, comprenant par exemple de l'aluminate de zinc, dans un milieu où l'on contrôle la teneur en eau à la fois au niveau de l'eau introduite par les réactifs et au niveau de l'eau qui s'accumule dans la boucle de recyclage du monoalcool aliphatique.

De façon plus détaillée, le procédé de l'invention comprend un ensemble de trois réactions équilibrées se déroulant en parallèle, et qui dans la suite du texte seront désignées sous le terme global de "la réaction":

### Réaction 1 :

L'huile (triglycéride) réagit avec une molécule de monoalcool (méthanol) pour donner une molécule d'ester alkylique et un diglycéride.

### Réaction 2 :

Le diglycéride réagit avec une molécule de monoalcool (méthanol) pour donner une molécule d'ester alkylique (méthylique) et un monoglycéride.

### Réaction 3 :

Le monoglycéride réagit avec une molécule de monoalcool (méthanol) pour donner une molécule d'ester alkylique (méthylique) et une molécule de glycérine.

Dans le cas où l'on cherche à produire du "Biodiesel", une des contraintes principales est la recherche d'une conversion poussée de l'huile vers la formation d'ester alkylique (méthylique) (96,5 % minimum), de façon à atteindre une teneur maximale en monoglycérides de 0,8 % en masse maximum. Cela implique de nombreuses contraintes sur le schéma de procédé, notamment un fort excès de monoalcool (méthanol) par rapport à la stoechiométrie, et au moins deux étapes réactionnelles entre lesquelles on élimine la glycérine produite de façon à déplacer l'équilibre vers la production d'ester alkylique (méthylique).

L'invention consiste donc en un procédé de production d'esters alkyliques d'acide gras et de glycérine à haute pureté, mettant en oeuvre un ensemble de réactions de transestérification entre une huile végétale ou animale et un monoalcool aliphatique, et utilisant un catalyseur hétérogène, comprenant par exemple de l'aluminate de zinc, caractérisé en ce que la teneur en eau dans le milieu réactionnel est contrôlée à une valeur inférieure à une valeur limite donnée. La teneur limite en eau dans le milieu réactionnel est généralement inférieure à 1500 ppm, et préférentiellement inférieure à 1000 ppm. L'ensemble des réactions de transestérification est généralement réalisé en au moins deux étapes, la première étape faisant réagir l'huile et le monoalcool dans une proportion de 20 à 80 % en masse pour l'huile, et préférentiellement dans une proportion de 45 à 55 % en masse pour la dite huile, et les étapes suivantes faisant réagir l'ester alkylique formé au cours de la première étape avec le monoalcool dans une proportion de 20 à 80 %, et préférentiellement de 45 à 55 % masse pour l'ester alkylique.

La réaction se fait en général en présence d'un catalyseur solide, par exemple comprenant de l'aluminate de zinc (comme décrit par exemple dans le brevet FR-B-2 752 242). L'avantage d'un catalyseur hétérogène sur les catalyseurs basiques homogènes classiques tels que la soude est qu'il permet d'éviter les nombreuses étapes de purification des produits formés qui contiennent la totalité du catalyseur. On évite donc aussi les rejets et leur traitement. Par exemple, la glycérine est ici exempte de sels et est pure à au moins 95 % et de préférence à 98 %. Dans le présent procédé hétérogène, il n'y a aucun effluent pollué rejeté.

La réaction se fait en général dans un ou plusieurs réacteurs en lit fixe successifs opérés en écoulement ascendant et en phase liquide, chacun des réacteurs étant alimenté par un mélange d'huile (par exemple de colza) et de monoalcool (par exemple méthanol) (premier réacteur) ou majoritairement d'ester alkylique (méthylique) et de monoalcool (méthanol) (second réacteur et éventuellement les réacteurs suivants). La proportion d'huile (par exemple de colza) ou d'ester alkylique (méthylique) est de 20 à 80 % et de préférence de 45 à 55 % en masse à l'entrée de chaque réacteur. Les conditions opératoires optimales se situent dans les fourchettes suivantes : de 30.10⁵ à 80.10⁵ Pa et préférentiellement de 40.10⁵ à 70.10⁵ Pa pour la pression, et de 453 à 493 °K, et préférentiellement de 463 à 483 °K pour la température. La teneur en eau dans le milieu réactionnel de chacun des réacteurs est contrôlée pour rester inférieure par exemple à 1500 ppm, et préférentiellement inférieure à 1000 ppm en masse. En sortie du ou des réacteurs, on obtient l'ester alkylique (par exemple méthylique) et un coproduit de la réaction, la glycérine, ainsi que le monoalcool aliphatique (par exemple méthanol) en excès. L'avantage du procédé est qu'après évaporation du monoalcool (méthanol) et séparation de l'ester alkylique (méthylique) et de la glycérine par décantation, on obtient des produits très purs et facilement valorisables.

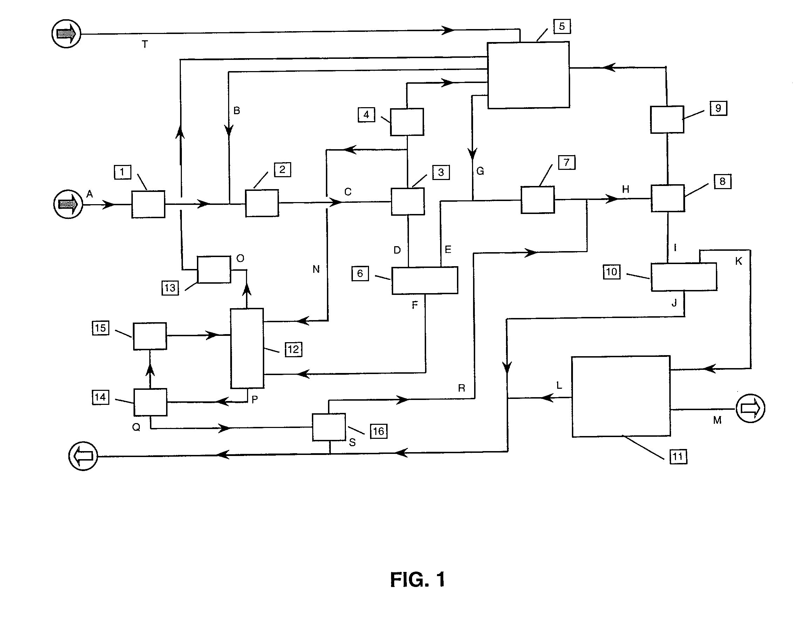

L'invention sera mieux comprise en suivant la Figure 1. Dans le procédé décrit plus particulièrement le monoalcool utilisé est le méthanol.

Selon le mode préféré de l'invention représenté, l'huile à traiter, ou huile de charge, provient généralement d'une unité de trituration de graines de colza ou d'autres huiles végétales comme par exemple l'huile de palme, de tournesol, de soja, de coprah, de coton ou de ricin, ainsi que des huiles d'origine animales comme le suif. On fait passer l'huile brute (A) dans un sécheur sous vide (1) de façon à obtenir une teneur en eau inférieure à 700 ppm en masse. On appelle dans la suite du texte "huile séchée" l'huile de charge ayant subi ce traitement.

L'huile séchée est mélangée avec du méthanol de recycle (B). Le mélange obtenu contenant de 20 à 80 %, et de préférence de 45 à 55 % en masse d'huile est comprimé à une pression par exemple de 62.10⁵ Pa et chauffé à une température par exemple de 473 °K (483 °K en fin de vie du catalyseur), passe de bas en haut dans un réacteur tubulaire (2) contenant un lit fixe d'un catalyseur comprenant de l'aluminate de zinc sous forme d'extrudés. La VVH, c'est-à-dire le rapport entre le débit volumique horaire d'huile à traiter et le volume de catalyseur est de 1,2 h-1 à 0,1 h-1 et préférentiellement de 0,6 à 0,4 h-1.

La conversion en huile obtenue dans ces conditions est d'au moins 90 % en masse, généralement d'au moins 92 % en masse. En sortie du réacteur (2), le mélange (C) contient de l'ester méthylique, de la glycérine, du méthanol et les glycérides peu ou pas convertis (huile, di- et monoglycérides). Ce mélange subit une phase de détente, puis d'évaporation du méthanol en excès dans un évaporateur (3) à une pression par exemple voisine de 2,5.10⁵ Pa. Le méthanol vapeur est condensé dans un condenseur (4) et recyclé vers le ballon tampon (5). Cette étape d'évaporation se fait de telle sorte que la teneur en méthanol résiduel dans le mélange soit de 5 à 25 % en masse, de préférence de 10 à 20 % en masse.

La présence dans le mélange d'une certaine teneur en méthanol est importante car celui-ci agit comme un co-solubilisant de l'ester méthylique et de la glycérine, naturellement insolubles. Le liquide (D) est alors refroidi jusqu'à 323 °K et décanté dans un ballon décanteur (6) pour séparer la phase supérieure (E), riche en ester méthylique alimentant la seconde section réactionnelle et la phase inférieure (F), riche en glycérine qui doit être traitée spécifiquement.

On rajoute à la phase ester méthylique issue du ballon de décantation (6) du méthanol issu du ballon tampon (5) de façon à obtenir à nouveau un mélange dont la teneur massique en ester méthylique est de 20 à 80 % et de préférence de 45 à 55 %. On fait passer le mélange obtenu de bas en haut dans un second réacteur (7) identique au premier, et opérant dans des conditions très sensiblement voisines de celles du réacteur (2). Dans la plupart des cas, les conditions opératoires des réacteurs (2) et (7) seront pratiquement identiques, et le catalyseur utilisé dans chacun des réacteurs peut être de même nature. La conversion obtenue à l'issue du réacteur (7) permet de satisfaire la spécification en mono-glycérides dans l'ester méthylique (H), qui est au maximum de 0,8 % en masse.

Le méthanol contenu dans le mélange des effluents issus du réacteur (7) est évaporé en deux étapes dans un ensemble d'évaporateurs (8).

La première étape d'évaporation se fait en général sdans les mêmes conditions que celle effectuée dans l'évaporateur (3), et la seconde étape d'évaporation est réalisée sous vide de façon à ne laisser qu'au maximum 500 ppm masse de méthanol dans le liquide (I), et de préférence 200 ppm, ce qui permet le séchage de l'ester méthylique à 200 ppm masse d'eau maximum. On obtient alors, après refroidissement et décantation de l'effluent lourd issu du groupe d'évaporateurs (8) dans le décanteur (10), une phase glycérine (J) très pure, qui peut partir directement en limite d'unité et une phase ester méthylique (K), qui subit un traitement décrit ci-dessous. Le méthanol vapeur issu de l'ensemble d'évaporateurs (8) est condensé dans le condenseur (9), puis recyclé vers le ballon tampon (5).

L'ester méthylique brut (K) issu du décanteur (10) peut être traité de façon à répondre à la spécification concernant la teneur en glycérine totale (libre et potentielle) qui est au maximum de 0,25 % en masse.

Ce traitement de l'ester méthylique brut peut se faire de différentes façons :

Par exemple, on peut faire passer l'ester méthylique dans un coalesceur (11) qui élimine les dernières traces de glycérine libre, puis éventuellement sur des masses adsorbantes, généralement des résines échangeuses d'ions, qui fixent la glycérine dissoute, dans un adsorbeur non représenté sur la Figure 1. La glycérine (L), très pure, séparée de l'ester méthylique, est envoyée en limite d'unité. L'ester méthylique final (M) part en limite d'unité.

Le traitement de l'ester méthylique peut dans d'autres cas se faire grâce à une ou plusieurs étapes de lavage à l'eau de l'ester.

Le flux de glycérine (F) issu du ballon décanteur (6), faisant partie de la première section réactionnelle, doit être traité de façon à atteindre une teneur maximum en méthanol de 5000 ppm en masse et une teneur maximale en MONG ("Matières Organiques Non-Glycérineuses") de 1 % en masse, qui correspondent au niveau commercial généralement acceptable.

L'évaporation du méthanol contenu dans le flux (F) se fait généralement en deux étapes. La première étape est réalisée en fond de la colonne de distillation (12). Cette colonne (12) assure deux services :
- l'évaporation du méthanol de la glycérine de fond jusqu'à une teneur de 5 % en masse de méthanol ; et
- la séparation eau/méthanol sur les plateaux de tête. Le méthanol de tête contient au maximum 800 ppm masse d'eau et de préférence 500 ppm.

Cette colonne est également alimentée par un flux (N) de méthanol vapeur venant de l'évaporateur (3) placé en aval du premier réacteur (2). Le méthanol (O) sortant en tête de la colonne (12) contient au maximum 800 ppm masse d'eau et de préférence 500 ppm. Le flux de méthanol (O) est condensé dans le condenseur (13), puis renvoyé vers le ballon tampon (5). Cette opération est nécessaire pour déconcentrer l'eau qui entre dans l'unité par l'huile de charge (A), dont le séchage est limité à 500 ppm masse, car pour pousser plus loin cette étape, il faut soit augmenter le niveau de vide, ce qui est coûteux, soit augmenter la température et risquer ainsi de décomposer une partie de l'huile. L'autre entrée d'eau provient du méthanol frais (T). En utilisant le méthanol commercial le plus sec, c'est à dire le grade A, on s'assure une teneur en eau inférieure à 1000 ppm masse. L'eau entrant dans le système par ces deux voies s'accumule dans la boucle méthanol. Comme il a été dit en introduction, l'eau est un inhibiteur du catalyseur, et au-delà d'une teneur en eau de 1000 ppm masse dans le mélange réactionnel, la conversion de l'huile chute sensiblement.

La glycérine (P) extraite du fond de la colonne (12) contenant environ 5 % de méthanol, est envoyée dans l'évaporateur sous vide (14). Le méthanol vapeur est condensé dans un condenseur (15) et recyclé vers la colonne (12). Le flux de glycérine (Q) extrait de l'évaporateur (14) contenant environ 3000 ppm de méthanol, est envoyé dans un ballon de décantation (16). La phase ester méthylique (R) issu de la tête du ballon décanteur (16) est renvoyée à l'entrée du groupe d'évaporateurs (8) faisant partie de la deuxième section réactionnelle et la glycérine purifiée (S) part en limite d'unité.

La réaction de transestérification consommant une partie du méthanol, il est nécessaire d'introduire du méthanol frais (T) dans le système.

Une partie de ce méthanol frais est envoyée dans le bac de charge de méthanol (5), l'autre partie peut servir à la régénération des résines échangeuses d'ions non représentée sur la Figure 1 au niveau du traitement de l'ester méthylique. Pour régénérer les résines saturées en glycérine, on utilise généralement un flux de méthanol pur. Ce méthanol souillé par de la glycérine et un peu d'ester méthylique est recyclé dans le procédé en amont du traitement de la glycérine. Puis on passe sur les résines régénérées un flux d'ester méthylique pur provenant du stockage de produit fini. L'ester méthylique souillé par du méthanol resté adsorbé sur les résines est recyclé au niveau de l'évaporation de la deuxième section réactionnelle.

### EXEMPLE

L'huile utilisée est de l'huile de colza et le méthanol est du méthanol commercial de grade A.

Dans le premier réacteur la conversion est définie comme la quantité d'huile ayant réagi, c'est-à-dire l'huile totalement convertie en ester méthylique (quantité d'huile totale moins la quantité de monoglycérides, diglycérides et de triglycérides) sur la quantité d'huile totale, soit : [50 - (1,8 + 1,0+ 0,6)]/50 = 93,2 %. La teneur en eau dans le premier réacteur est de 1000 ppm.

Dans le deuxième réacteur, on continue de rapporter la conversion à la quantité d'huile entrant dans le premier réacteur et en tenant compte des monoglycérides et des diglycérides ayant réagi dans le second réacteur. La conversion est alors de : [50 - (0,3 + 0,1 + 0)]/50 = 99,2 %. La teneur en eau dans le deuxième réacteur est de 1200 ppm.

Pour les deux étapes réactionnelles, les conditions opératoires sont identiques. Le rapport massique méthanol sur huile est de 50/50 dans le premier réacteur et le rapport massique méthanol sur ester méthylique est de 48/49,6 dans le deuxième réacteur.

La température est de 473 °K. La pression est de 62.10⁵ Pa. La VVH huile/catalyseur est de 0,5 h⁻¹.

Le Tableau 1 ci-après fournit un bilan matière autour de la première et de la deuxième zone réactionnelle.

**Tableau 1 :**

| **bilan matière dans la zone réactionnelle** | | | | |
|---|---|---|---|---|
| Débit (kg/h) | Entrée Réacteur 1 | Sortie Réacteur 1 | Entrée Réacteur 2 | Sortie Réacteur 2 |
| Méthanol | 50,0 | 44,8 | 50,0 | 48,0 |
| Glycérine | 0,0 | 4,5 | 0,5 | 1,0 |
| Ester méthylique | 0,0 | 47,3 | 46,2 | 49,0 |
| Monoglycérides | 0,0 | 1,8 | 1,8 | 0,8 |
| Diglycérides | 0,0 | 1,0 | 1,0 | 0,2 |
| Huile | 50,0 | 0,6 | 0,5 | 0,0 |
| Total | 100,0 | 100,0 | 100,0 | 100,0 |

Pour illustrer l'intérêt de travailler en milieu à teneur en eau contrôlée, le Tableau 2 ci-après montre, en sortie de la deuxième étape réactionnelle, la conversion telle que définie précédemment en fonction de la teneur en eau en ppm dans le milieu réactionnel. On constate très clairement que la teneur en eau dans le milieu réactionnel influe directement et de manière très sensible sur la conversion. Avec une teneur en eau inférieure à 1500 ppm, et de préférence inférieure à 1000 ppm dans le milieu réactionnel, la conversion se situe à des niveaux très élevés, supérieurs à 99,2 %.

**Tableau 2 :**

| **influence de la teneur en eau dans la charge sur la conversion en ester méthylique (2**^{**ème**} **étape de catalyse)** | |
|---|---|
| Teneur en eau dans la charge (ppm) | Conversion en ester (% masse) |
| 400 | 99,4 |
| 100 | 99,25 |
| 2500 | 98,9 |
| 5000 | 98,6 |
| 8000 | 98,5 |

## Revendications

1. Procédé de production d'esters alkyliques d'acide gras et de glycérine à haute pureté, mettant en oeuvre un ensemble de réactions de transestérification entre une huile végétale ou animale et un monoalcool aliphatique et utilisant un catalyseur hétérogène **caractérisé en ce que** la teneur en eau dans le milieu réactionnel, est contrôlée à une valeur inférieure à une valeur limite donnée.

2. Procédé selon la revendication 1 dans lequel le catalyseur comprend de l'aluminate de zinc.

3. Procédé selon la revendication 1 ou 2 dans lequel la teneur en eau dans le milieu réactionnel est inférieure à 1500 ppm.

4. Procédé selon la revendication 1 ou 2 dans lequel la teneur en eau dans le milieu réactionnel est inférieure à 1000 ppm.

5. Procédé selon la revendication 1 à 4 dans lequel l'ensemble des réactions de transestérification est réalisé en au moins deux étapes, la première étape faisant réagir l'huile végétale ou animale et le monoalcool dans une proportion de 20 à 80 % en masse pour l'huile et les étapes suivantes faisant réagir l'ester alkylique formé à l'issue de la première étape avec le monoalcool dans une proportion de 20 à 80 % en masse pour l'ester alkylique.

6. Procédé selon la revendication 1 à 4 dans lequel l'ensemble des réactions de transestérification est réalisé en au moins deux étapes, la première étape faisant réagir l'huile végétale ou animale et le monoalcool dans une proportion de 45 à 55 % en masse pour l'huile et les étapes suivantes faisant réagir l'ester alkylique formé à l'issue de la première étape avec le monoalcool dans une proportion de 45 à 55 % en masse pour l'ester alkylique.

7. Procédé selon l'une des revendications 1 à 6 dans lequel chacune des étapes réactionnelles se déroule à une pression de 30.10⁵ Pa à 80.10⁵ Pa et à une température de 453 à 493 °K et à une VVH de 1,2 à 0,1 h⁻¹.

8. Procédé selon l'une des revendications 1 à 6 dans lequel chacune des étapes réactionnelles se déroule à une pression de 40.10⁵ à 70.10⁵ Pa et à une température de 463 à 483 °K et à une VVH de 0,6 à 0,4 h⁻¹.

9. Procédé selon l'une des revendications 1 à 8 dans lequel la glycérine obtenue est exempte de sels et possède une pureté d'au moins 95 % et, de façon préférée, d'au moins 98 %.

10. Procédé selon l'une quelconque des revendications 1 à 9 dans lequel l'huile de charge est une huile végétale provenant d'une unité de trituration d'huile de colza, de palme, de tournesol, de soja, de coprah, de coton ou de ricin, ou consiste en suif.

11. Procédé selon l'une des revendications 1 à 10 dans lequel l'ester alkylique issu de la dernière étape réactionnelle fait l'objet d'une séparation sur des masses adsorbantes régénérables telles que des résines échangeuses d'ions, en vue de le purifier de la glycérine qu'il contient.

12. Procédé selon la revendication 11 dans lequel la séparation est effectuée sur des résines échangeuses d'ions.

13. Procédé selon l'une des revendications 1 à 10 dans lequel l'ester alkylique obtenu à l'issue de la dernière étape réactionnelle fait l'objet de plusieurs étapes de lavage à l'eau en vue de le purifier de la glycérine qu'il contient.
